(19) Europäisches Patentamt
European Patent Office
Office européen des brevets

(11) **EP 3 070 011 B1**

(12) **EUROPEAN PATENT SPECIFICATION**

(45) Date of publication and mention
of the grant of the patent:
**11.08.2021 Bulletin 2021/32**

(51) Int Cl.:
***A61L 2/28*** *(2006.01)*

(21) Application number: **14859580.4**

(22) Date of filing: **09.10.2014**

(86) International application number:
**PCT/JP2014/005149**

(87) International publication number:
**WO 2015/068334 (14.05.2015 Gazette 2015/19)**

(54) **STERILIZATION DEVICE FOR CONTAINER**

STERILISATIONSVORRICHTUNG FÜR BEHÄLTER

DISPOSITIF DE STÉRILISATION POUR RÉCIPIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.11.2013 JP 2013232663**

(43) Date of publication of application:
**21.09.2016 Bulletin 2016/38**

(73) Proprietor: **Mitsubishi Heavy Industries Machinery Systems, Ltd.**
**Hyogo-ku, Kobe-shi**
**Hyogo 652-8585 (JP)**

(72) Inventors:
• **TANAKA, Daisuke**
**Tokyo 108-8215 (JP)**
• **YUI, Sugihiko**
**Nagoya-shi**
**Aichi 453-0862 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 279 952          EP-A1- 2 772 447**
**WO-A1-2004/045784     DE-A1- 4 114 889**
**DE-A1-102012 103 023   JP-A- H0 947 494**
**JP-A- S55 104 670         JP-A- 2001 004 556**
**JP-A- 2004 099 111        JP-A- 2005 176 864**
**JP-A- 2008 044 640        JP-A- 2008 044 640**
**JP-A- 2010 155 631        JP-A- 2013 216 391**
**JP-A- 2013 216 391        US-A1- 2007 253 863**
**US-A1- 2014 144 105**

• **None**

## Description

Technical Field

The present invention relates to a sterilization device for a beverage container, and particularly to a sterilization device for a PET bottle (a plastic bottle).

Background Art

To aseptically fill a container, for example, with a tea, a fruit beverage or a coffee beverage, the inside and outside of the container have to be sterilized before filling. Thus, a paper container or a plastic container such as a PET bottle (hereinafter referred to as beverage container) is sterilized using an aseptic filling system.

In such an aseptic filling system, sterilization is performed with the beverage container being at an increased temperature in order to improve the effect of a sterilizing agent (for example, JP 2010-155631 A). The sterilizing agent supplied in a gas or mist form is harder to adhere to a portion having a relatively high surface temperature. Thus, variation in surface temperature of the beverage container leads to adhesion in a higher amount to a portion having a relatively low surface temperature, thereby causing local unevenness of sterilization.

Moreover, EP 2 279 952 A1 discloses a drink filling method comprising the steps of: blasting hydrogen peroxide mist or gas into a container in an erected attitude through a mouth portion thereof; activating the hydrogen peroxide in the container in an inverted attitude by blasting heated aseptic air through the mouth portion thereof directed downward, and simultaneously, removing foreign substance from the container by replacing air in the container with the aseptic air; filling the container in the erected attitude with drink through the mouth portion directed upward; and closing the mouth portion of the container by applying a lid to the mouth portion of the container.

Other related art may be found in WO 2004/045784 A1 describing a machine for rinsing and/or sterilizing bottles made of plastic or glass, EP 2 772 447 A1 being directed to a beverage filling method and apparatus and DE 10 2012 103023 A1 disclosing a device for sterilizing containers. Further related art may be found in US 2014/144105 A1, DE 41 14 889 A1, US 2007/253863 A1, JP 2013-216391 A and JP 2008-044640 A. JP2008044640 discloses a sterilisation device for bottles comprising a sterilant spraying device, a control system, a laser beam and a camera sensor to evaluate the amount of sterilant on the bottle after sterilization, and determine if it has been sufficiently sterilized or should be submitted to further treatment.

In the sterilization processing, a sterilizing agent supply pipe is fixed at a distance from the mouth of the beverage container or is inserted into the beverage container, before the sterilizing agent is injected in the beverage container. The injected sterilizing agent hits on the bottom of the beverage container, and adheres to inner walls of the beverage container while going up toward the mouth. The remaining sterilizing agent, which has not adhered to the beverage container, exhausts from the mouth to the exterior of the beverage container.

A narrow diameter of the mouth of the beverage container accelerates the sterilizing agent passing through the mouth, which reduces the sterilizing agent that may adhere to the mouth. In addition, the sterilizing agent has been consumed due to adhesion to the inner surfaces of the beverage container until the sterilizing agent reaches the mouth. Therefore, the mouth essentially has a tendency to be less susceptible to sterilization.

Summary of Invention

Technical Problem

The problem with the mouth being less susceptible to sterilization can be avoided with excess injection of the sterilizing agent. The excess injection, however, leads to an excess amount of the sterilizing agent near the bottom of the container and to increased cost of the beverage.

The present invention addresses the above problem. An object of the present invention is to provide a sterilization device for adhering an appropriate amount of a sterilizing agent to a portion that is less susceptible to adhesion of the sterilizing agent.

Solution to Problem

The present invention is defined by the appended independent claim 1. The respective dependent claims describe optional features and preferred embodiments.

To achieve the object, the present invention provides a sterilization device, wherein the sterilization device includes sterilizing agent supply part for supplying a sterilizing agent into a container having a mouth and a shoulder continuous with the mouth through the mouth, wherein sterilizing agent detecting means including a light source and an optical sensor having a light receiving element configured to detect a level of the sterilizing agent adhering to the shoulder of the container, to which the sterilizing agent is supplied, by irradiating the shoulder with inspection light, and control means for determining an amount of the sterilizing agent to be supplied to the container in the sterilizing agent supply means based on the result of the detection by the sterilizing agent detecting means.

A not claimed aspect of present invention provides a sterilization device, wherein the sterilization device includes temperature detecting means for detecting a high temperature position having a relatively high temperature in a container after the container is preheated, sterilizing agent supply means for supplying a sterilizing agent into the container after the high temperature position is detected, sterilizing agent measuring means for measuring an adhesion amount of the sterilizing agent adhering to the high temperature position after the sterilizing agent is supplied, and control means for determining an amount of the sterilizing agent to be supplied to the container in the sterilizing agent supply means in accordance with the adhesion amount of the sterilizing agent in the high temperature position.

A further not claimed aspect of present invention provides a sterilization device, wherein the sterilization device includes sterilizing agent supply means for supplying a sterilizing agent into a container, imaging means for imaging the sterilizing agent adhering to the container after the sterilizing agent is supplied, and control means for determining an amount of the sterilizing agent to be supplied to the container in the sterilizing agent supply means in accordance with data imaged by the imaging means.

Advantageous Effects of Invention

According to the present invention, a container such as a beverage container can be sterilized with an appropriate amount of a sterilizing agent. Therefore, excess supply of the sterilizing agent to the container can be avoided.

Brief Description of Drawings

[FIG. 1] FIG. 1 is a view of a schematic configuration of an aseptic beverage filling apparatus of the present invention.
[FIG. 2] FIG. 2A and FIG. 2B are views of a sterilization device for a container that is adapted to the aseptic beverage filling apparatus in FIG. 1.
[FIG. 3] FIG. 3 is a view of an evaporation device for evaporating the sterilizing agent.
[FIG. 4] FIG. 4 is a view of a sterilization device in the first embodiment.
[FIG. 5] FIG. 5 is a view of a positional relationship between a light source and a sensor in the first embodiment.
[FIG. 6] FIG. 6 is a view of a sterilization device in the second embodiment.
[FIG. 7] FIG. 7 is a view of a positional relationship between light sources and sensors in the second embodiment.
[FIG. 8] FIG. 8 is a view of a sterilization device in the third embodiment.
[FIG. 9] FIG. 9A and FIG. 9B are views of a container whose adhesion state of a sterilizing agent is imaged by imaging means.

Description of Embodiments

The first to third embodiments according to the present invention will be described below. These embodiments have some common parts in the basic device configuration, and thus the basic configuration will be first described.

The following describes a sterilization device of the present invention based on one embodiment in which the sterilization device is adapted to an aseptic beverage filling apparatus.

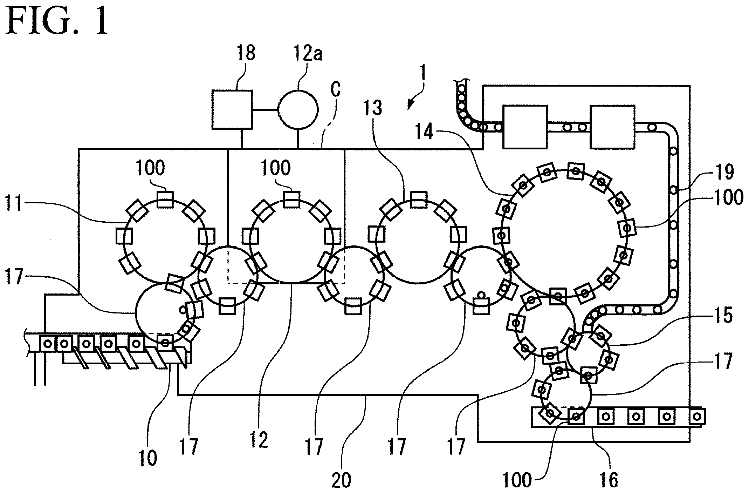

As illustrated FIG. 1, an aseptic beverage filling apparatus 1 includes a carrying-in conveyor 10 for carrying containers 100 in the aseptic beverage filling apparatus 1, a preheating device 11 for preheating the containers 100, a sterilization device 12 for sterilizing the containers 100, a rinse device 13 for rinsing the containers 100, a filling device 14 for filling the sterilized and rinsed containers 100 with a liquid (a beverage), a capper 15 for attaching a cap 19 to each container 100 filled with the beverage, and a carrying-out conveyor 16 for carrying the containers 100 out of the aseptic beverage filling apparatus 1. Between these components, carrying star wheels 17 are provided to deliver the containers 100 between the components. The container 100 may include a plastic bottle, for example.

A carrying path for the containers 100 in the preheating device 11, the sterilization device 12, the rinse device 13, the filling device 14, and the capper 15 is provided on a base pedestal 20. On the base pedestal 20, a chamber C that covers the sides and the top of the space on or above the base pedestal 20 is provided to maintain the carrying path for the containers 100 in an aseptic environment.

In the chamber C, shower-like or sprinkler-like spraying nozzles (not shown) are provided to spray a sterilizing agent or a rinsing liquid into the chamber C.

In the base pedestal 20, drain ports (not shown) for recovering the sprayed sterilizing agent or the rinsing liquid are also formed. The recovered sterilizing agent or the like is exhausted through the drain port to an external recovering part.

The preheating device 11 preheats the container 100 to a predetermined temperature, before the sterilizing agent is sprayed in the sterilization device 12. Preheating the container 100 can improve the sterilization effect for the container 100.

Preheating is performed by injecting hot gas from a hot-gas supply pipe (not shown) inserted into the container 100 and convecting the hot gas in the container 100.

The temperature of the hot gas to be injected is not less than 40°C to achieve the sterilization effect.

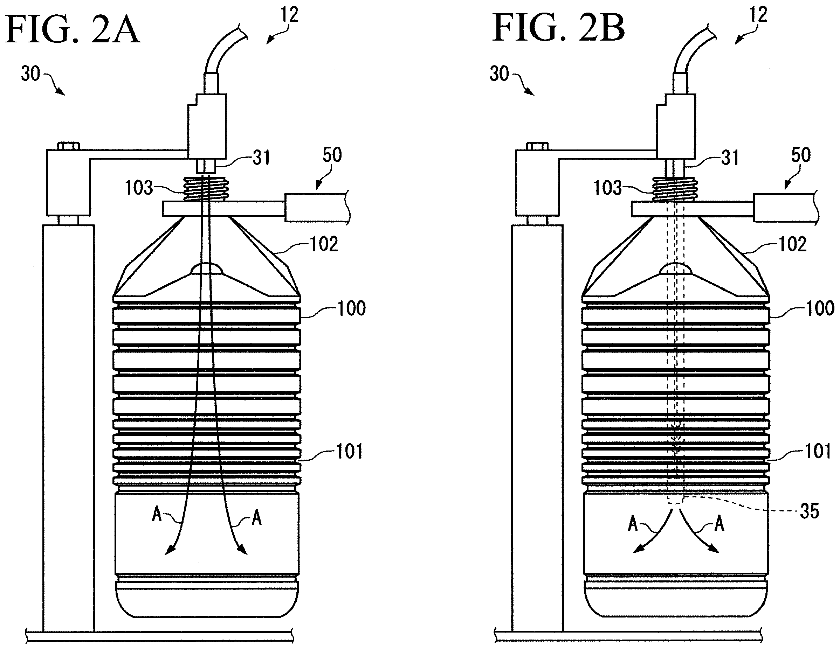

As illustrated in FIGS. 2A and 2B, the sterilization device 12 supplies the sterilizing agent to the container 100 to sterilize the inner surfaces thereof, while the container 100 supported by a container supporting part 50 is carried. The sterilization device 12 includes a sterilizing agent supply part 30 and the container supporting part 50. The sterilization device 12 is also provided with a sterilizing agent supply tank 12a.

The sterilizing agent supply tank 12a supplies the sterilizing agent to the chamber C and to the sterilizing agent supply part 30 in the sterilization device 12. The sterilizing agent supplied to the chamber C is used for sterilizing the inside of the chamber C, while the sterilizing agent supplied to the sterilization device 12 is used for sterilizing the inside and outside of the container 100. Wide variety of chemicals, for example peracetic acid or hydrogen peroxide, may be used as the sterilizing agent.

As illustrated in FIG. 2A, the sterilizing agent supply part 30 provides sterilization in the following steps: a sterilizing agent supply pipe 31 is fixed near the mouth 103 of the container 100 in a non-contact state; and then the sterilizing agent is supplied to the sterilizing agent supply pipe 31 and injected from the tip of the sterilizing agent supply pipe 31 toward the bottom of the container 100 so that the sterilizing agent adheres to the inner surfaces of the container 100. Alternatively as illustrated in FIG. 2B, the sterilizing agent supply part 30 provides sterilization in the following steps: the sterilizing agent supply pipe 31 is inserted into the container 100; and then the sterilizing agent is supplied to the sterilizing agent supply pipe 31 and injected from the tip of the sterilizing agent supply pipe 31 (arrows A) so that the sterilizing agent adheres to the inner surfaces of the container 100.

The sterilizing agent supply pipe 31 is in a hollow and cylindrical form, and has an injection nozzle 35 formed at its end for injecting the sterilizing agent. The sterilizing agent supply pipe 31 is preferably made of a metal material, for example a stainless steel excellent in corrosion resistance. Materials other than metal materials, for example ceramic materials, may be used.

In the sterilization process, the above situation of the container 100, in which the mouth 103 is directed upward (in FIGS. 2A and 2B), is only an example. The mouth 103 may be directed downward, sideward or in an inclined direction. The container 100 may be sterilized while being rotated or swung.

The sterilizing agent used in the present invention is evaporated or converted into mist for sterilizing the container 100.

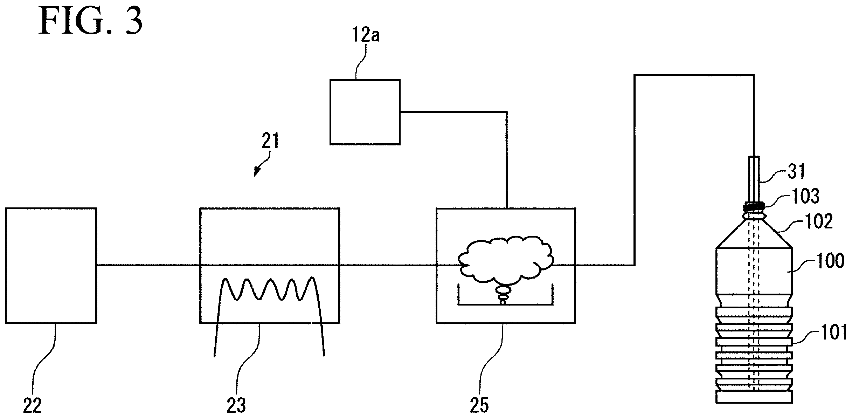

As illustrated in FIG. 3, the sterilizing agent is evaporated by an evaporation device 21 before being supplied to the sterilizing agent supply pipe 31.

The evaporation device 21 includes an air blower 22, a heater 23, and an evaporator 25, which are connected via piping in this order.

The heater 23 heats the air blown from the air blower 22 and sends the heated air into the evaporator 25. The sterilizing agent is also supplied to the evaporator 25 from the sterilizing agent supply tank 12a.

Consequently, the sterilizing agent is heated by the blown hot air, and is evaporated or converted into mist. The sterilizing agent evaporated or converted into mist is fed from the evaporator 25 to the sterilizing agent supply pipe 31, and is then injected from the injection nozzle 35 into the container 100 at 40°C to 70°C.

A control device 18 provides an instruction to the sterilizing agent supply tank 12a in order to adjust the amount of the sterilizing agent to be supplied to the container 100. The control device 18 can also specify the amount of the sterilizing agent adhering to the shoulder 102, for example in accordance with an electrical signal from an optical sensor 45. The control device 18 can also calculate a deficient amount of the sterilizing agent.

[First Embodiment]

In the first embodiment, the amount of the sterilizing agent adhering to the shoulder 102 of the container 100 is measured in accordance with light transparency to inject an appropriate amount of the sterilizing agent.

Essentially, the amount of the sterilizing agent in the mouth 103 should be measured in order to supply the required amount of the sterilizing agent to the mouth 103. However, light transparency or absorbance in the mouth 103 is not stable, since the mouth 103 is thicker than the shoulder 102 and a main body 101 and is threaded to be closed with a cap. The shoulder 102 is located adjacent to the mouth 103, and thus the adhesion amount of the sterilizing agent in the shoulder 102 relatively approximates to the adhesion amount of the sterilizing agent in the mouth 103. Therefore in this embodiment, the adhesion amount of the sterilizing agent in the shoulder 102 is measured, and the adhesion level of the sterilizing agent in the mouth 103 is estimated using the result of the measurement.

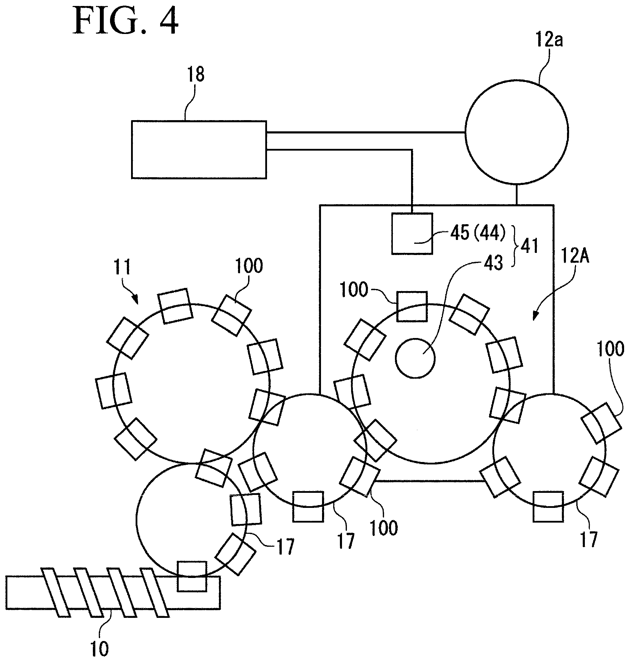

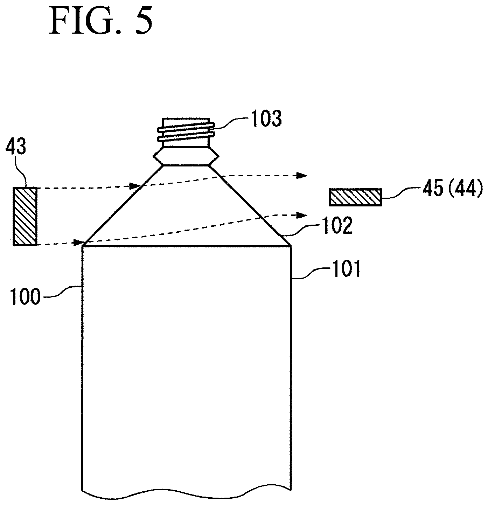

This embodiment is further described below with reference to FIG. 4 and FIG. 5. In the description, a sterilization device 12A characteristic of the first embodiment is first described, and a process related to sterilization of the container 100 is next described.

The sterilization process is typically performed for preparing to fill the first container 100 with a beverage. The same statement is true for the second and third embodiments.

[Sterilization Device 12A]

The sterilization device 12A is provided with a sterilizing agent measuring device (sterilizing agent detecting means) 41, as illustrated in FIG. 4. The sterilizing agent measuring device 41 includes a light source 43 and an optical sensor 45 having a light receiving element 44.

The light receiving element 44 is connected to the control device 18, and is arranged across the container 100 from the light source 43. The optical sensor 45 is positioned so that it is irradiated by the light source 43 and the light receiving element can detect the inspection light passing through the shoulder 102 of the container 100.

The amount of light detected by the light receiving element 44 varies with the amount of the sterilizing agent adhering to the shoulder 102. The light receiving element 44 has a photoelectric conversion feature to send an electrical signal corresponding to an intensity of the received light to the control device 18. The control device 18 specifies the amount of the sterilizing agent adhering to the shoulder 102 in accordance with the electrical signal received from the light receiving element 44, as described in detail hereinafter.

The light source 43 uses visible light having a center wavelength ranging from 400 nm to 600 nm.

The container 100 is heated by the preheating device 11 as a pretreatment for sterilizing the container 100.

The preheating device 11 injects hot gas to heat the container 100.

A hot gas supply pipe, which is not shown, is inserted from the mouth 103 into the container 100, and hot gas is then injected near the bottom of the container 100. The injected hot gas passes through the inside of the container 100, in order of the main body 101, the shoulder 102, and the mouth 103, and exhausts out of the container 100. The hot gas heats the inside of the container 100 between the injection and the exhaust, which warms up the container 100.

The heated container 100 is carried to the sterilization device 12A by the carrying star wheels 17, as illustrated in FIG. 4.

The sterilizing agent supply pipe 31 is fixed to the container 100 carried in the sterilization device 12A in an appropriate relative position. The sterilizing agent evaporated or converted into mist is then injected from the injection nozzle 35 toward the bottom of the container 100.

The injected sterilizing agent moves from the bottom of the main body 101 and exhausts out of the container 100. Between the injection and the exhaust, the sterilizing agent adheres to the inner surfaces of the main body 101, the shoulder 102, and the mouth 103 in this order, and, as a result, sterilizes the container 100.

After the injection of the sterilizing agent, the sterilizing agent measuring device 41 measures the amount of the sterilizing agent adhering to the shoulder 102 of the container 100.

Since the mouth 103 opens to the outside and is narrower than the main body 101, the sterilizing agent passing through the mouth 103 is accelerated. Thus, the sterilizing agent is hard to adhere to the inner surfaces of the mouth 103. In this point, most of the sterilizing agent has been consumed due to adhesion to the main body 101, and the sterilizing agent that may reach the mouth 103 has been reduced. For the above reasons, the mouth 103 is least susceptible to sterilization in the container 100.

As described above, it is difficult for an optical sensor, for example, to accurately detect the amount of light transmitted through the mouth 103. In this embodiment, the sterilizing agent measuring device 41 therefore targets the shoulder 102 that is located near the mouth 103. The sterilizing agent measuring device 41 measures the amount of light transmitted through the shoulder 102, which is represented by $V_{11}$. The sterilizing agent measuring device 41 then sends an electric signal related to the amount of light $V_{11}$ to the control device 18. The control device 18 determines an amount of the sterilizing agent to be supplied to the container 100 in the sterilizing agent supply part 30, based on the result of the detection by the sterilizing agent measuring device 41.

The control device 18 in the first embodiment stores data related to the injection amount of the sterilizing agent required for reliable sterilization of the mouth 103. This data, i.e. a prescribed amount, is represented by $V_{10}$.

When the control device 18 receives an amount of light $V_{11}$ from the sterilizing agent measuring device 41, the control device 18 determines the difference $\Delta V$ between the amount of light $V_{11}$ and the prescribed amount $V_{10}$ (formula 1) .

$$\Delta V = V_{10} - V_{11} \cdots (1)$$

If $\Delta V$ is a negative value, the control device 18 determines that the adhesion amount of the sterilizing agent is in a low level, and specifies the amount of the sterilizing agent to be increased in accordance with $\Delta V$. On the other hand, if $\Delta V$ is a positive value, the control device 18 determines that the adhesion amount of the sterilizing agent is in a high level, and specifies the amount of the sterilizing agent to be decreased in accordance with $\Delta V$. For specifying the amount of the sterilizing agent to be increased or decreased, the control device 18 may store table-type data and check $\Delta V$ against the data, for example.

The control device 18 provides an instruction to the sterilizing agent supply tank 12a to operate so that the sterilizing agent in an amount in accordance with the specified amount is injected.

The sterilizing agent supply tank 12a then increases or decreases the amount of the sterilizing agent to be supplied to the evaporator 25 under the instruction. Consequently, the sterilizing agent is injected from the injection nozzle 35 so that an appropriate amount of the sterilizing agent adheres to the mouth 103.

In the case of $\Delta V=0$, the sterilizing agent is not increased or decreased. $V_{10}$ is not limited to a specific value and may have a range of values. A mechanism, in which a product that gives $\Delta V$ deviating from a prescribed range of $V_{10}$ is treated as a non-standard and removed the product from the production line, may be provided.

In the sterilization device 12A of this embodiment, the adhesion amount of the sterilizing agent in the shoulder 102 is measured. Although this is an indirect way, the adhesion amount of the sterilizing agent in the mouth 103 can be calculated accurately. Only when the adhesion amount in the mouth 103 is determined to be lower than the prescribed amount, the sterilizing agent to be supplied to the container 100 is increased by the required amount. On the other hand, when the adhesion amount in the mouth 103 is determined to be higher than the prescribed amount, the sterilizing agent to be supplied to the container 100 is decreased by an appropriate amount.

Therefore, it is not necessary to supply an excess amount of the sterilizing agent to the container 100, and the sterilization level of the mouth 103 can be improved with the sterilizing agent supplied in an appropriate minimum amount.

This embodiment may be applied at any timing. For example, measurement may be performed at an early stage of a series of continuous beverage filling processes for the containers 100 (batch processing), and the result of the measurement may be reflected to the subsequent stage of the filling processes. Alternatively, the measurement may be performed on a prior batch processing, and the result of the measurement on the prior batch processing may be reflected after the prior batch processing ends and before the next batch processing begins.

[Second Embodiment]

The shape of the container has been complicated with trends in the type of beverage to be filled or the appearance of the container. Thus, the single aseptic beverage filling apparatus 1 may be used for filling different shaped containers with a beverage.

For some shapes of containers, the mouth 103 does not necessarily have the lowest adhesion amount of the sterilizing agent in the container.

In the container 100, the injected sterilizing agent is easier to adhere to a portion having a lower temperature, and is harder to adhere to a portion having a higher temperature.

Thus, in the second embodiment, a position, in which an adhesion amount of the sterilizing agent is to be measured, is determined based on the temperature distribution in the preheated container 100, and the amount of the sterilizing agent to be injected is then determined in accordance with the adhesion amount of the sterilizing agent in the position.

In the description of this embodiment, the container 100 having a similar shape to the container of the first embodiment is used for convenience.

[Sterilization Device 12B]

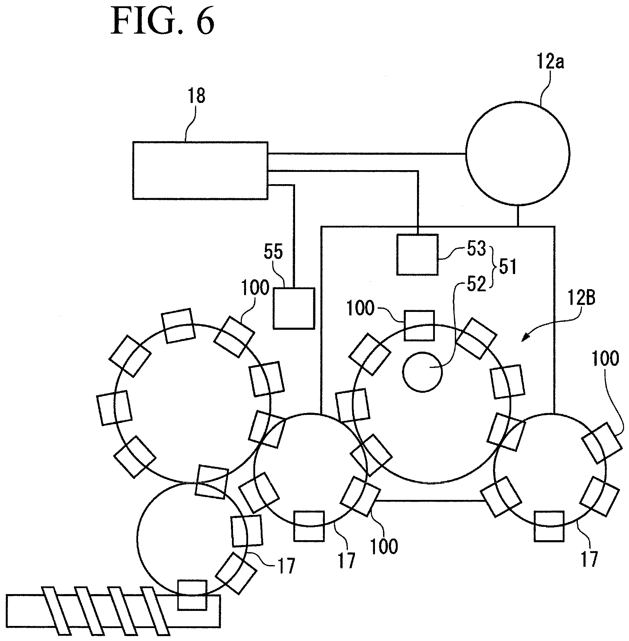

A sterilization device 12B is provided with a sterilizing agent measuring device 51, as illustrated in FIG. 6.

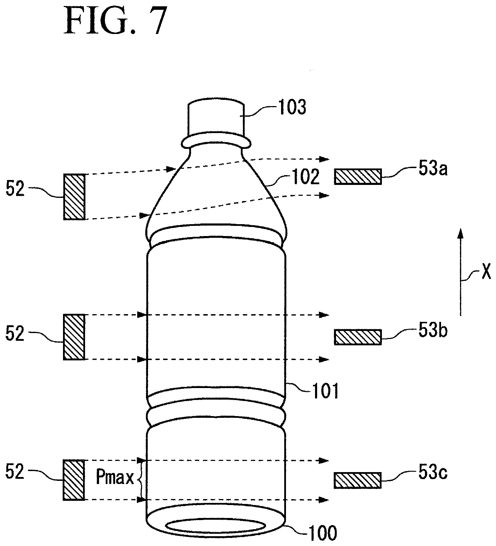

The sterilizing agent measuring device 51 includes light sources 52, and a plurality of sensors 53 (53a to 53c) arranged in the height direction X of the container 100, as illustrated in FIG. 7.

Each sensor 53 can detect an adhesion amount of the sterilizing agent adhering to a predetermined region. For example, the sensor 53c can detect the adhesion amount near the bottom of the container 100. An optical sensor, for example, can be used to detect the adhesion amount of the sterilizing agent.

A sterilization process is now described. Since the procedure for preheating the container 100 and the procedure for supplying the sterilizing agent are similar to those of the first embodiment, the following description is focused on differences from first embodiment for the sake of simplicity.

Temperature detecting means 55 determines the temperature distribution in the entire preheated container 100 positioned in a predetermined position. Infrared thermography or a thermal image measuring device can be used as the temperature detecting means 55, for example. The temperature detecting means 55 detects the portion (position) having the highest temperature in the container 100 (hereinafter referred to as $P_{MAX}$) The position near the bottom corresponds to $P_{MAX}$ in this embodiment.

The temperature detecting means 55 sends information about $P_{MAX}$ to the control device 18.

Although $P_{MAX}$ in the container 100 may be detected after the container 100 is preheated, $P_{MAX}$ is preferably measured immediately before the container 100 is carried to the sterilization device 12B.

The control device 18 sends information about the position $P_{MAX}$ to the sterilizing agent measuring device 51. The sterilizing agent measuring device 51 measures the adhesion amount of the sterilizing agent $V_{21}$ in the position $P_{MAX,}$ i.e. near the bottom of the container 100, based on the information, and then sends information about the adhesion amount $V_{21}$ to the control device 18.

The control device 18 stores information about the prescribed amount $V_{20}$ for sterilizing the container 100.

The control device 18 checks the adhesion amount $V_{21}$ against the prescribed amount $V_{20}$. If the adhesion amount $V_{21}$ is lower than the amount required for sterilization (the prescribed amount $V_{20}$), the control device 18 calculates the deficient amount of the sterilizing agent. The control device 18 then provides an instruction to the sterilizing agent supply tank 12a to operate so that the sterilizing agent is increased by the calculated amount. The sterilizing agent supply tank 12a received the instruction feeds the appropriate amount of the sterilizing agent into the evaporator 25.

In this way, the amount of the sterilizing agent to be supplied is determined, and the aseptic beverage filling apparatus 1 is then brought into operation.

According to the sterilization device 12B of this embodiment, the sterilizing agent measuring device 51 detects the adhesion amount of the sterilizing agent in the position having a relatively high temperature in the container 100, and the control device 18 then determines the amount of the sterilizing agent to be supplied by the sterilizing agent supply part 30 so that the prescribed amount of the sterilizing agent adheres to the position. Consequently, the container 100 can be sterilized with an appropriate minimum amount of the sterilizing agent.

As mentioned above, even if the container 100 has a complex shape, the inside of the container 100 can be sterilized with an appropriate amount of the sterilizing agent.

[Third Embodiment]

In the third embodiment, an appropriate amount of the sterilizing agent to be injected is determined through observation of the entire container 100 with the sterilizing agent adhering thereto.

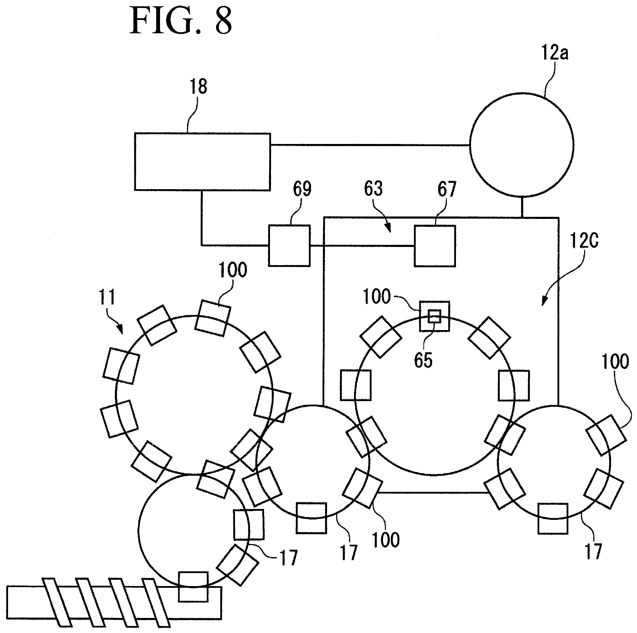

A sterilization device 12C of this embodiment is provided with imaging means 63, as illustrated in FIG. 8.

The imaging means 63 includes a light source 65 for irradiating the mouth 103 of the container 100 with light, a camera 67 for taking an image of the container 100 irradiated with the light, and a display part 69 for displaying the image taken by the camera 67. For example, a CCD camera can be used as the camera 67.

When the container 100 is irradiated with light from the light source 65, the adhering sterilizing agent is scattered. Consequently, a luminance of a portion with the sterilizing agent adhering thereto is higher than a luminance of a portion with no sterilizing agent. Therefore, it is recognized that a portion with a higher luminance has a relatively high adhesion amount of the sterilizing agent compared to a portion with a lower luminance.

An image of the container 100 is taken by the camera 67 while the container 100 is irradiated with the light, and the taken image is displayed on the display part 69.

A sterilization process of this embodiment is now described.

The container 100 is heated by the preheating device 11. The heated container 100 is carried to the sterilization device 12C by the carrying star wheels 17. The sterilizing agent is then injected from the injection nozzle of the sterilizing agent supply pipe 31 toward the bottom of the container 100.

After the injection of the sterilizing agent, the image of the container 100 with the sterilizing agent adhering thereto is displayed on the display part 69 by the imaging means 63. The adhesion amount of the sterilizing agent can be determined from the luminance of the image.

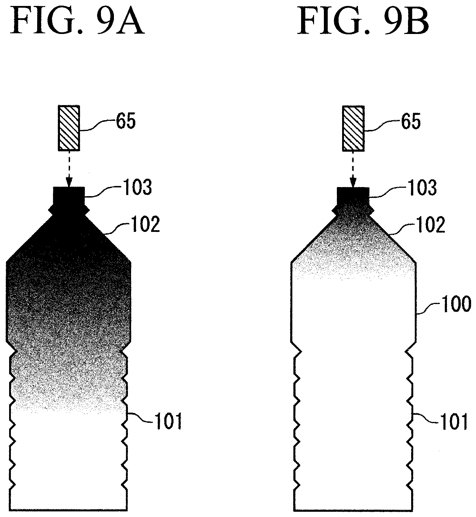

For example in FIG. 9A, the luminance decreases from the bottom to the mouth 103, and scattering is not observed in the shoulder 102 and the mouth 103. Thus, it is recognized that substantially no sterilizing agent adheres to the shoulder 102 and the mouth 103. On the other hand, FIG. 9 shows that the luminance is high throughout the entire container 100. Thus, it is recognized that the sterilizing agent substantially entirely adheres to the container 100.

Since a luminance in an image results from scattering caused by the sterilizing agent, there is a correlation between the adhesion amount of the sterilizing agent and the luminance of the image.

The control device 18 digitizes information about a luminance magnitude (threshold) that is shown when the prescribed amount $V_{30}$ of the sterilizing agent adheres, and stores the digitized information in advance. The control device 18 analyzes the entirety of the image of the container 100, and determines if the luminance reaches the threshold. If a portion having a luminance lower than the threshold is found in the image of the container 100, the control device 18 instructs the sterilizing agent supply tank 12a to increase the amount of the sterilizing agent to be supplied to the evaporator 25.

In this way, the amount of the sterilizing agent to be supplied is determined by the control device 18, and the aseptic beverage filling apparatus 1 is then brought into operation.

According to the sterilization device 12C of this embodiment, an operator can visually determine the adhesion level of the sterilizing agent throughout the entire container 100, and thus easily determine if the container 100 is entirely sterilized.

If a portion having an insufficient adhesion amount of the sterilizing agent is detected, the control device 18 operates to increase the amount of the sterilizing agent to be supplied by the required amount. Consequently, it is not necessary to supply an excess amount of the sterilizing agent, and the sterilization can be performed with an appropriate minimum amount of the sterilizing agent.

Although sterilization, which is described as an example in the embodiments, is performed as a preparation to operate the aseptic beverage filling apparatus 1, sterilization may be performed during the operation of the aseptic beverage filling apparatus 1 in practice.

In the third embodiment, the sterilizing agent may be mixed with a pigment to determine the adhesion state of the sterilizing agent from color of the pigment.

Reference Signs List

    1 aseptic beverage filling apparatus
    10 carrying-in conveyor
    11 preheating device
    12 sterilization device
    12A, 12B, 12C sterilization device
    12a sterilizing agent supply tank
    13 rinse device
    14 filling device
    15 capper
    16 carrying-out conveyor
    17 carrying star wheel
    18 control device

19 cap
20 base pedestal
21 evaporation device
22 air blower
23 heater
25 evaporator
30 sterilizing agent supply part
31 sterilizing agent supply pipe 35 injection nozzle
41 sterilizing agent measuring device (sterilizing agent detecting means)
43 light source
45 sensor
50 container supporting part
51 sterilizing agent measuring device
52 light source
53 sensor
55 temperature detecting means 63 imaging means
65 light source
67 camera
69 display part
100 container
101 main body
102 shoulder
103 mouth

**Claims**

1. A sterilization device (12) for a container (100), comprising a sterilizing agent supply part (30) configured for supplying a sterilizing agent into said container (100) having a mouth (103) and a shoulder (102) continuous with said mouth (103) said sterilizing agent being supplied through said mouth (103), and sterilizing agent detecting means (41) including a light source (43) configured to irradiate the shoulder (102) of the container(100) with inspection light and an optical sensor (45) having a light receiving element (44) configured to detect a level of the sterilizing agent adhering to the shoulder (102) of the container (100), to which the sterilizing agent is supplied; Said optical sensor (45) and said light source (43) being positioned so that the light receiving element can detect the inspection light passing through the shoulder (102) of the container (100),
**characterized by**
control means for determining an amount of the sterilizing agent to be supplied to said container (100) in the sterilizing agent supply part based on the result of the detection by the sterilizing agent detecting means (41).

2. The sterilization device (12) for a container (100) according to claim 1, wherein said container (100) is a beverage container.

3. The sterilization device (12) for a container (100) according to claim 2, wherein said beverage container is made of plastic.

4. The sterilization device (12) for a container (100) according to any one of claims 1 to 3, wherein the sterilizing agent supply part (30) is configured to supply the sterilizing agent into said container (100), wherein the sterilizing agent being evaporated or converted into mist.

**Patentansprüche**

1. Sterilisationsvorrichtung (12) für einen Behälter (100), die umfasst
einen Sterilisationsmittelzuführteil (30), der dazu konfiguriert ist, ein Sterilisationsmittel in den Behälter (100) zuzuführen, der eine Mündung (103) und eine Schulter (102), die mit der Mündung (103) kontinuierlich ist, aufweist, wobei das Sterilisationsmittel durch die Mündung (103) zugeführt wird, und Sterilisationsmittelerfassungsmittel (41), die eine Lichtquelle (43), die dazu konfiguriert ist, die Schulter (102) des Behälters (100) mit Inspektionslicht zu bestrahlen, und einen optischen Sensor (45), der ein Lichtempfangselement (44) aufweist, beinhalten, der dazu konfiguriert ist, ein Niveau des Sterilisationsmittels, das an der Schulter (102) des Behälters (100) haftet, dem das Sterilisationsmittel zugeführt wird, zu erfassen;
wobei der optische Sensor (45) und die Lichtquelle (43) derart positioniert sind, dass das Lichtempfangselement das Inspektionslicht, das durch die Schulter (102) des Behälters (100) durchgeht, erfassen kann,
**gekennzeichnet durch**
Steuermittel zum Bestimmen einer Menge des Sterilisationsmittels, die dem Behälter (100) in dem Sterilisationsmittelzuführteil basierend auf dem Resultat der Erfassung durch das Sterilisationsmittelerfassungsmittel (41) zugeführt werden soll.

2. Sterilisationsvorrichtung (12) für einen Behälter (100) nach Anspruch 1, wobei der Behälter (100) ein Getränkebehälter ist.

3. Sterilisationsvorrichtung (12) für einen Behälter (100) nach Anspruch 2, wobei der Getränkebehälter aus Kunststoff hergestellt ist.

4. Sterilisationsvorrichtung (12) für einen Behälter (100) nach einem der Ansprüche 1 bis 3, wobei der Sterilisationsmittelzuführteil (30) dazu konfiguriert ist, das Sterilisationsmittel in den Behälter (100) zuzuführen, wobei das Sterilisationsmittel verdampft oder in einen Nebel umgewandelt wird.

**Revendications**

1. Dispositif de stérilisation (12) pour un récipient (100), comprenant
   une partie d'alimentation en agent de stérilisation (30) configurée pour alimenter un agent de stérilisation dans ledit récipient (100) ayant un goulot (103) et un épaulement (102) dans la continuité dudit goulot (103), ledit agent de stérilisation étant alimenté à travers ledit goulot (103), et
   des moyens de détection d'agent de stérilisation (41) incluant une source lumineuse (43) configurée pour irradier l'épaulement (102) du récipient (100) avec une lumière d'inspection et un capteur optique (45) présentant un élément de réception de lumière (44) configuré pour détecter un niveau de l'agent de stérilisation adhérant à l'épaulement (102) du récipient (100), vers lequel l'agent de stérilisation est alimenté ; ledit capteur optique (45) et ladite source lumineuse (43) étant positionnés de sorte que l'élément de réception de lumière puisse détecter la lumière d'inspection passant à travers l'épaulement (102) du récipient (100),
   **caractérisé par**
   des moyens de commande pour déterminer une quantité de l'agent de stérilisation à alimenter vers ledit récipient (100) dans la partie d'alimentation d'agent de stérilisation sur la base du résultat de la détection par les moyens de détection d'agent de stérilisation (41).

2. Dispositif de stérilisation (12) pour un récipient (100) selon la revendication 1, dans lequel ledit récipient (100) est un récipient de boisson.

3. Dispositif de stérilisation (12) pour un récipient (100) selon la revendication 2, dans lequel ledit récipient de boisson est réalisé en plastique.

4. Dispositif de stérilisation (12) pour un récipient (100) selon l'une quelconque des revendications 1 à 3, dans lequel la partie d'alimentation d'agent de stérilisation (30) est configurée pour alimenter l'agent de stérilisation dans ledit récipient (100), dans lequel l'agent de stérilisation est évaporé ou converti en brouillard.

# FIG. 1

FIG. 2B

FIG. 2A

# FIG. 3

# FIG. 4

# FIG. 5

FIG. 6

# FIG. 7

# FIG. 8

# FIG. 9A

# FIG. 9B

**EP 3 070 011 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010155631 A
- EP 2279952 A1
- WO 2004045784 A1
- EP 2772447 A1
- DE 102012103023 A1
- US 2014144105 A1
- DE 4114889 A1
- US 2007253863 A1
- JP 2013216391 A
- JP 2008044640 A
- JP 2008044640 B